# EUROPÄISCHE PATENTSCHRIFT

(11) **EP 0 272 601 B1**
(45) Veröffentlichungstag und Bekanntmachung des Hinweises auf die Patenterteilung: **17.02.1993**
(21) Anmeldenummer: 87118658.1
(22) Anmeldetag: 16.12.1987
(51) Int. Cl.: A61H 33/02

(54) **Badewanne mit Luftsprudelsystem**
Bath with an air bubble system
Baignoire avec un système à injection d'air

(30) Priorität: 23.12.1986 DE 3644109
(43) Veröffentlichungstag der Anmeldung: 29.06.1988
(73) Patentinhaber: HOESCH Metall + Kunststoffwerk GmbH & Co., 52304 Düren (DE)
(72) Erfinder: Klotzbach, Manfred, D-5160 Düren (DE)
(74) Vertreter: Langmaack, Jürgen, Dipl.-Ing.

(56) Entgegenhaltungen:
- EP-A- 0 224 615
- EP-A- 0 268 050
- WO-A-86/01100
- DE-A- 2 951 091
- DE-A- 3 447 161
- DE-A- 3 447 772
- DE-A- 3 607 788
- GB-A- 875 477
- GB-A- 2 176 101
- US-A- 4 000 528
- US-A- 4 170 044

## Beschreibung

Die Erfindung betrifft eine Badewanne mit einem verschließbaren Wannenablauf, der an ein Wannenablaufrohr angeschlossen ist, und mit vorzugsweise im Bodenbereich eingebauten Düsen für die Einleitung von Luft, mit denen die Druckseite eines Gebläses über wenigstens eine Luftzuleitung verbunden ist, wobei in der Zuleitung zwischen Gebläse und der, in Strömungsrichtung der Luft gesehen, ersten Düse ein Rückflußverhinderer angeordnet ist.

Die Anwendung des Luftsprudelbades erfolgt heute über den medizinisch-therapeutischen Bereich hinaus in zunehmendem Maße auch im privaten Bereich, so daß seitens der Benutzer der Wunsch nach einfacher Bedienung besteht. Insbesondere muß aber dafür Sorge getragen werden, daß die Wanne so ausgebildet ist, daß auch eine unter Hygienegesichtspunkten einwandfreie Reinigung des Rohrsystems in einfacher Weise möglich ist. Dies ist bei der aus GB-A-875 477 bekannten Wanne mit schalenförmiger Luftzuleitung nicht gegeben.

Der Erfindung liegt nun die Aufgabe zugrunde, eine Badewanne der vorstehend bezeichneten Art so auszubilden, daß eine zuverlässige und einfach zu handhabende desinfizierende Reinigung durchgeführt werden kann.

Diese Aufgabe wird erfindungsgemäß dadurch gelöst, daß die Luftzuleitung als Rohrleitung ausgebildet und mit dem Wannenablaufrohr verbunden ist und daß - bezogen auf die Luftbeaufschlagung - zwischen der letzten Düse und der Einmündung in das Wannenablaufrohr in der Rohrleitung ein mit einem unabhängig vom Wannenablauf steuerbaren Stellantrieb versehenes Absperrventil angeordnet ist, daß in die Rohrleitung eine Zuleitung für eine Spülflüssigkeit einmündet, die an eine Spülflüssigkeitsversorgung angeschlossen ist und daß in der Zuleitung - in Strömungsrichtung gesehen - hinter der Einmündung der Spülflüssigkeitsversorgung ein Rückflußverhinderer angeordnet ist. Da nach einer Benutzung vor der Entleerung der Wanne das Gebläse abgeschaltet wird, dringt über die Luftdüsen der Wanneninhalt auch in die Rohrleitung für die Luftzufuhr ein. Durch die absperrbare Verbindung der Rohrleitung mit dem Wannenablaufrohr ist nun sichergestellt, daß nach vollständiger Entleerung der Wanne auch das in die Rohrleitung eingedrungene Wasser über das Wannenablaufrohr abgeführt werden kann. Um nun zu verhindern, daß die im benutzten Wasser enthaltenen Keime in der an sich nicht zugänglichen Rohrleitung Keimherde bilden können, kann nun bei dieser Anordnung nach vollständiger Entleerung der Rohrleitung in einfacher Weise auch eine Desinfektion durchgeführt werden. Hierzu wird dann der Wannenablauf geschlossen und bei geöffnetem Absperrventil die Rohrleitung mit Frischwasser zunächst durchgespült. In einem nächsten Schritt wird dann das Absperrventil geschlossen und erneut bei geschlossenem Wannenablauf mit einer Desinfektionslösung versetztes Wasser über die Luftdüsen in die Rohrleitung eingebracht, so daß diese nach einer kurzen Einwirkungszeit keimfrei ist und nach erneutem Öffnen des Absperrventils die Rohrleitung vollständig entleert werden kann. Zweckmäßig ist es, für das Absperrventil ein Magnetventil zu verwenden, das in seinem Steuerantrieb so ausgebildet ist, daß es "stromlos offen" ist, so daß bei Nichtbenutzung das Absperrventil geöffnet bleibt und die Rohrleitung über die Luftdüsen einerseits und die Wannenablauföffnung andererseits mit der Umgebungsluft in Verbindung bleibt und trocknen kann. Zugleich ist gewährleistet, daß bei einem geringen Wasserzulauf über einen undichten oder nicht ganz geschlossenen Wannenhahn das in die Wanne einlaufende Wasser immer abfließen kann. Wird der Rücklaufverhinderer in nur kurzem Abstand zur ersten Düse angeordnet, so ist sichergestellt, daß nur geringe Wassermengen zum Reinigen benötigt werden. Zum anderen ist sichergestellt, daß bei einem im Bereich des Wannenkastens angeordneten Gebläse kein Wasser in das Gebläse eintreten kann. Die erfindungsgemäße Anordnung hat auch den Vorteil, daß mit einer vorbereiteten, mit Desinfektionsmitteln versehenen Spülflüssigkeit die Reinigung der Rohrleitungen zu den Luftdüsen vorgenommen werden kann. Der besondere Vorteil besteht hierbei darin, daß nach dem Ablauf der benutzten Wannenfüllung aus der Wanne und der Rohrleitung zu den Luftdüsen das Absperrventil geschlossen werden kann und bei geöffnetem Wannenablauf die Rohrleitung mit der Spülflüssigkeit nach Art einer "Rückspülung" zwangsweise durchspült werden kann, so daß neben der chemischen Einwirkung durch die Spülfüssigkeit auch eine mechanische Reinigung über den Wasserstrom erfolgt.

Zweckmäßigerweise ist in Ausgestaltung der Erfindung für die Versorgung mit Spülflüssigkeit ein Speicherbehälter vorgesehen. Dieser kann in der einfachsten Form als Hochbehälter ausgeführt werden, so daß über das Gefälle der erforderliche Spüldruck aufgebracht wird. In zweckmäßiger weiterer Ausgestaltung der Erfindung ist jedoch vorgesehen, daß in der vom Speicherbehälter ausgehenden Zuleitung für die Spülflüssigkeit eine Pumpe angeordnet ist. Dies erlaubt zum einen eine sehr vielkompaktere Bauweise und erlaubt es, die Spülflüssigkeit mit höherem Druck in die Rohrleitung einzubringen, so daß der mechanische Spüleffekt verbessert wird. Wird bei Einleitung des Spülvorganges zunächst das Absperrventil offengehalten, dann können mit der Spülflüssigkeit zunächst etwaige Ablagerungen aus der Rohrleitung direkt in das Wannenablaufrohr ausgespült werden, so daß für den nachfolgenden "Desinfektionsvorgang" bei geschlossenem Absperrventil die "Rückspülung" keine Ablagerungen mehr durch die Luftdüsen in den Wanneninnenraum fördern kann. Zweckmäßigerweise ist auch in der Zuleitung für die Spülflüssigkeit ein Rückflußverhinderer angeordnet.

Da als Spülflüssigkeit in der Regel Wasser mit einem Desinfektionszusatz verwendet wird, ist in zweckmäßiger Ausgestaltung der Erfindung vorgesehen, daß der Speicherbehälter an eine Wasserzuleitung angeschlossen ist, die über ein Schwimmerventil absperrbar ist. Dies hat den Vorteil, daß der Speicherbehälter immer gefüllt ist, was insbesondere bei einer integrierten Steuerschaltung, wie nachstehend in weiterer Ausgestaltung der Erfindung noch dargelegt werden wird, die Gesamtanordnung ohne laufende Kontrolle des Spülflüssigkeitsvorrates betreibbar ist. Das Desinfektionsmittel kann hierbei automatisch in Abhängigkeit von dem Wasserzulauf zudosiert werden oder aber in gewissen Zeitabständen über Dosiertabletten eingebracht werden. Besonders zweckmäßig ist es, wenn als Schwimmerventil ein Spülkastenventil verwendet wird. Unter Spülkastenventil ist ein Sperrventil zu verstehen, wie es bei Toilettenspülkästen eingesetzt wird. Der Vorteil besteht nicht nur darin, daß hier ein handelsübliches Bauelement eingesetzt werden kann, sondern daß darüber hinaus aufgrund der strikten Prüfungs- und Zulassungsvorschriften für derartige Bauelemente auch für die Anwendung auch in diesem Bereich auf ein zugelassenes Bauteil zurückgegriffen werden kann. Zweckmäßigerweise ist der Speicherbehälter mit einem Überlaufrohr versehen, das an das Wannenablaufrohr angeschlossen ist. Hierdurch ist sichergestellt, daß bei einem Defekt des Schwimmerventils das überschüssige Wasser fortlaufend abgeführt werden kann.

In weiterer vorteilhafter Ausgestaltung der Erfindung ist vorgesehen, daß an der Wand der Wanne mit Abstand über dem Boden ein Niveaufühler angeordnet ist, der über eine Steuerschaltung mit dem Gebläse verbunden ist, die ein Einschalten erst dann zuläßt, wenn das durch den Niveaufühler vorgegebene Betriebsniveau erreicht ist. Derartige Niveaufühler für den Einbau in Badewannen sind grundsätzlich bekannt. Sie können sowohl in direktem Kontakt, d.h. als innenliegende Kontaktelektroden ausgebildet sein, als auch kapazitiv ausgebildet sein, d.h. durch auf der Wannenaußenseite in entsprechender Höhe angeordnete Metallstreifen gebildet werden. Durch diese Anordnung ist sichergestellt, daß die Beaufschlagung der Luftdüsen nicht vor einer vollständigen Füllung der Wanne erfolgen kann. Wird nämlich das Gebläse bei nur geringer Wasserfüllung eingeschaltet, würde die mit einem auf das Betriebsniveau abgestellten Druck einströmende Luft erhebliche Wassermengen hochreißen und in den Raum verspritzen.

In weiterer Ausgestaltung der Erfindung ist vorgesehen, daß an der Wand der Wanne unterhalb des ersten Niveaufühlers ein zweiter Niveaufühler mit geringem Abstand über dem Wannenboden angeordnet ist, der über eine Steuerschaltung mit dem Stellantrieb des Absperrventils verbunden ist, die das Absperrventil beim Befüllen erst dann schließt, wenn das durch den zweiten Niveaufühler vorgegebene Mindestniveau in der Wanne erreicht ist. Hierdurch wird eine "Systemvorspülung" in der Weise erreicht, daß beim Befüllen der Wanne ein Teil des frisch zulaufenden Wassers bei geöffnetem Absperrventil über die Luftdüsen und die damit verbundene Rohrleitung abläuft und hierbei die Rohrleitung des Luftsprudelsystems durchspült. Da der Gesamtdurchtrittsquerschnitt entweder der Luftdüsen oder des Rohrleitungsstückes zwischen Wannenablaufrohr und Absperrventil geringer ist als der maximale Zulaufquerschnitt der Badewannenmischarmatur, ist hierbei sichergestellt, daß nach einer relativ kurzen Zeit das Mindestniveau erreicht wird und das Absperrventil automatisch geschlossen wird.

In weiterer Ausgestaltung der Erfindung ist vorgesehen, daß das Absperrventil, die Spülpumpe und das Gebläse über ein Zeitschaltwerk in der Steuerschaltung so miteinander verbunden sind, daß beim Entleeren der Wanne bei Unterschreitung des Mindestniveaus bei geschlossenem Absperrventil nach Ablauf einer Zeitdauer t1 die Spülpumpe für eine Zeitdauer t2 eingeschaltet wird und nach Ablauf der Zeitdauer t2 während einer Zeitdauer t3 bei geschlossenem Absperrventil die Spülpumpe abgeschaltet bleibt, daß nach Ablauf der Zeitdauer t3 das Absperrventil öffnet und daß nach Ablauf einer Zeitdauer t4 das Absperrventil schließt und das Gebläse mit geringer Leistung einschaltet und daß nach Ablauf einer Zeitdauer t5 das Gebläse abschaltet und das Absperrventil öffnet. Durch eine derartige Verschaltung von Absperrventil, Spülpumpe und Gebläse wird ein automatischer Ablauf der Entleerung, Durchspülung und Reinigung der Rohrleitungen des Luftsprudelsystems erreicht. Die Zeitdauer t1 ist hierbei so bemessen, daß eine vollständige Entleerung der Wanne sichergestellt ist. Die Zeitdauer t2 ist so bemessen, daß bei geschlossenem Absperrventil und geöffnetem Wannenablauf die in der Rohrleitung noch enthaltene Brauchwassermenge und eine definierte Spülwassermenge durch die Rohrleitung hindurchgeführt wird, so daß sichergestellt ist, daß beim Abschalten der Spülpumpe die Rohrleitungen nur noch mit Spülflüssigkeit gefüllt sind. Die Zeitdauer t3 ist so bemessen, daß die bei dem jeweiligen Desinfektionsmittel notwendige Einwirkungszeit eingehalten ist. Erst nach Ablauf der Zeitdauer t3 öffnet das Absperrventil, so daß die Spülflüssigkeit abfließen kann. Die hierfür erforderliche Zeitdauer t4 ist so bemessen, daß ein vollständiger Ablauf der Spülflüssigkeit gewährleistet ist. Danach schließt das Absperrventil bei gleichzeitigem Einschalten des Gebläses mit geringer Leistung, d.h. mit niedriger Drehzahl, so daß über eine Zeitdauer t5 die Rohrleitung über die Luftdüsen "trockengeblasen" wird. Ggf. kann im Bereich der Luftzuführungsleitung auf der Druckseite des Gebläses noch eine zusätzliche Beheizungseinrichtung vorgesehen werden, so daß das Trockenblasen mit aufgeheizter Luft erfolgt. Mit Ablauf der Zeitdauer t5 schaltet die Steuerschaltung vollständig ab. Hierdurch wird das Absperrventil stromlos gesetzt und öffnet.

In Ausgestaltung der Erfindung ist für eine Badewanne aus Kunststoff, insbesondere aus Acryl, mit einer Wannenbodenverstärkung vorgesehen, daß die mit den Luftdüsen verbundene Rohrleitung im Bereich des Wannenbodens wenigstens teilweise in das Material der Wannenbodenverstärkung eingebettet ist. Dies hat den Vorteil, daß die im wesentlichen in Badewannenlängsrichtung verlaufenden Rohrleitungen in Verbindung mit der Wannenbodenverstärkung dieser die Struktur eines Hohl- bzw. Kastenträgers geben und damit wesentlich zu einer Versteifung der Wannenbodenverstärkung beitragen. Die Wannenbodenverstärkung kann hierbei beispielsweise in Form eines glasfaserverstärkten Polyester-Laminates aufgebracht werden. Besonders vorteilhaft und in der Verarbeitung einfacher ist es, wenn gemäß einer anderen Ausgestaltung die Wannenbodenverstärkung durch ein mit Füllstoffen vermischtes Gießharz, vorzugsweise Polyester, aufgebracht wird. Dadurch, daß die Rohrleitungen des Luftdüsensystems über die Wannenverstärkung in die Wanne integriert sind, ergibt sich eine Vereinfachung der Montage, da eine fertig vormontierte Badewanne zum Aufstellungsort transportiert werden kann und dort im Rahmen der ohnehin erforderlichen Installationsanschlußarbeiten montiert werden kann.

Gemäß einer anderen Ausgestaltung der Erfindung ist es auch möglich, daß die Wannenbodenverstärkung durch ein vorgefertigtes Formteil aus mit Füllstoffen versetztem Gießharz gebildet wird, in das die Rohrleitung mit den zugehörigen Anschlußteilen für die Düsen eingegossen wird und das vorzugsweise durch Klebung mit der Außenfläche des Wannenkörpers fest verbunden wird. Dies ist insbesondere für die Fertigung großer Stückzahlen von Vorteil, da dann in einem gesonderten Fertigungsvorgang die Wannenbodenverstärkung in Form eines "Brettes" hergestellt werden kann, auf das nach Auftrag eines Klebers der mit entsprechenden Bohrungen versehene Wannenkörper aufgesetzt und fest mit der Wannenbodenverstärkung verbunden werden kann. Da in der Regel die Düsen in die entsprechenden Anschlußteile eingeschraubt werden, kann hierüber die erforderliche Verpressung zwischen Wannenkörper und Wannenbodenverstärkung zur Verbesserung der Klebeverbindung bewerkstelligt werden. Gegenüber dem unmittelbaren Vergießen eines fertig montierten Systems auf dem Wannenboden liegt ein besonderer Vorteil darin, daß bei einer getrennten Fertigung der Wannenbodenverstärkung auf die Wärmeentwicklung beim Aushärten des Gießharzes keine Rücksicht genommen zu werden braucht und somit die Gefahr einer Beschädigung des in der Regel aus Acryl gezogenen Wannenkörpers durch diese Wärmeentwicklung ausgeschlossen ist.

In einer vorteilhaften anderen Ausgestaltung ist vorgesehen, daß die Wannenbodenverstärkung durch wenigstens eine Verstärkungsplatte, vorzugsweise aus einem Holzwerkstoff, gebildet wird, daß die Rohrleitung an deren Rand entlang verläuft und daß die Verstärkungsplatte mit der Rohrleitung in einer geschlossenen Schicht aus einem gießbaren aushärtenden Harz eingebettet ist, die im Randbereich umlaufend mit der Wannenaußenfläche verbunden ist. Hierdurch ergibt sich eine erhebliche Vereinfachung bei der Herstellung derartiger Wannen auf der Basis verhandener Wannenkörper ohne Luftsprudeleinrichtung.

In zweckmäßiger Weiterbildung der Erfindung ist vorgesehen, daß die Verstärkungsplatte auf ihrer der Wannenaußenfläche zugekehrten Seite mit jeweils einer Nut zur Aufnahme der Rohrleitung versehen ist, die mit Kleber ausgefüllt ist, der nach dem Andrücken der Verstärkungsplatte an die Wannenaußenfläche die Rohrleitung umschließt. Dies hat den Vorteil, daß die Rohrleitung, die beispielsweise bei einem Luftsprudelsystem einen verhältnismäßig geringen Außendurchmesser hat, von der Verstärkungsplatte abgedeckt ist, zugleich aber fest in diese durch den Kleber eingebunden ist. Außerdem wird die notwendige Harzmenge für die Abdeckung der Verstärkungsplatte reduziert, da es nunmehr ausreicht, die außenliegende Fläche der Verstärkungsplatte einschließlich der umlaufenden Seitenkanten mit einer dünnen Deckschicht aus Harz, beispielsweise im Spritzverfahren, zu versehen. Anstelle von Harz kann auch eine entsprechende wasserbeständige Farbe aufgetragen werden. Diese Deckschicht ist insbesondere bei der Verwendung einer Verstärkungsplatte aus einem Holzwerkstoff als Schutz gegen Verrottung durch Feuchtigkeitseinflüsse wichtig.

Die Erfindung wird anhand einer schematischen Zeichnung eines Ausführungsbeispieles näher erläutert. Es zeigen:
- Fig. 1: eine Wannenanordnung im Längsschnitt,
- Fig. 2: eine Wanne im Querschnitt.

Die schematisch dargestellte Badewanne 1 ist beispielsweise aus Acrylharz im Tiefziehverfahren hergestellt. Die Bodenfläche 2 der Badewanne 1 ist hierbei beispielsweise in zwei parallelen Reihen mit einer Vielzahl von Luftdüsen 3 versehen, die jeweils an eine gemeinsame Rohrleitung 4 angeschlossen sind, die auf der Außenseite des Wannenbodens in Wannenlängsrichtung verläuft und die Luftdüsen mit einem Gebläse 10 verbindet. Die Rohrleitung 4 ist in eine Wannenbodenverstärkung 5 aus einem mit Füllstoffen versetzten, aushärtenden gießbaren Harz eingebettet, die mit der Außenseite des Wannenbodens fest verbunden ist. Über einen Rückflußverhinderer 6 wird der Rücklauf von Wasser in Richtung auf das Gebläse 10 unterbunden. Das andere Ende der Rohrleitung 4 ist unter Zwischenschaltung eines von außen betätigbaren Absperrventils 7,beispielsweise eines Magnetventils,an den Siphon 8 des Badewannenablaufrohres 9 angeschlossen. Das Absperrventil 7 ist in einer entsprechenden Aussparung der Wannenverstärkung 5 angeordnet.

Ist die Wanne gefüllt und das Absperrventil 7 geschlossen, dann kann über das Gebläse 10 die Rohrleitung 4 mit Luft beaufschlagt werden, so daß aus den einzelnen Düsen 3 die Luft in einer Vielzahl von Blasen in die Wannenfüllung eintritt und den Körper des Badenden umspült. Zum Entleeren der Wanne kann nun der Wannenablauf 9ʹ geöffnet werden, wobei weiterhin die Luftversorgung solange eingeschaltet bleibt, bis die Wanne vollständig entleert ist. Wird jedoch die Luftversorgung vorher abgeschaltet, so läuft die Rohrleitung 4 bis zum Rückverhinderer 6 voll Wasser. Wird dann der Wannenablauf 9ʹ geöffnet, muß zusätzlich auch das Absperrventil 7 geöffnet werden, so daß die Rohrleitung 4 ebenfalls leerlaufen kann. Um die Ausbildung von Bakterienherden in der Rohrleitung 4 zu verhindern, wird zweckmäßigerweise nach Abschluß des Badevorganges der Wannenablauf 9ʹ geschlossen und die Wanne einschließlich der Rohrleitung 4 bei geöffnetem Absperrventil 7 mit Frischwasser, ggf. unter Zugabe eines Desinfektionsmittels, über die Düsen 3 gespült.

In die Rohrleitung 4 mündet - in Strömungsrichtung der aus dem Gebläse 10 kommenden Luft gesehen - vor der ersten Düse eine Zuleitung 11 für eine Spülflüssigkeit ein, die an eine Versorgungseinheit für eine Spül- und Desinfektionsflüssigkeit angeschlossen ist. Bei dem dargestellten Ausführungsbeispiel besteht die Versorgungseinheit aus einem Speicherbehälter 12, der über ein Schwimmerventil 13 mit einer Wasserzuleitung 14 in Verbindung steht. Als Schwimmerventil ist hierbei ein sogenanntes Spülkastenventil eingesetzt, das als handelsübliches und vor allem von den Wasserversorgungsunternehmen zugelassenes und erprobtes Bauteil für den Einbau an dieser Stelle vorzuziehen ist. Durch eine mit dem Wannenablaufrohr 9 verbundene Überlaufleitung 15 ist sichergestellt, daß bei einem Defekt des Schwimmerventils der Wasserzulauf auch fortlaufend abgeführt wird.

Der Speicherbehälter 12 ist auslaufseitig mit einer Spülpumpe 16 versehen, an die die mit der Rohrleitung 4 verbundene Zuleitung 11 unter Zwischenschaltung eines Rückflußverhinderers 17 angeschlossen ist. Das Desinfektionsmittel kann beispielsweise über eine sogenannte Dauertablette im Speicherbehälter oder über eine gesonderte, entsprechend angesteuerte Dosierpumpe in die Wasserfüllung des Speicherbehälters 12 eingegeben werden.

An einer Wand der Wanne ist mit Abstand über dem Boden ein Niveaufühler 18 angeordnet, durch den das Betriebsniveau, d.h. die vollständige für ein Sprudelbad vorgesehene Badewannenfüllung definiert ist. Unterhalb des Niveaufühlers 18 ist ein zweiter Niveaufühler 19 mit geringem Abstand über dem Wannenboden 2 angeordnet, durch den ein Mindestniveau in derWanne definiert wird. Beide Niveaufühler sind mit einer nachstehend noch näher zu erläuternden Steuerschaltung verbunden.

Grundsätzlich ist es möglich, die einzelnen Funktionen gesondert von Hand zu schalten, wobei u.U. zwischen den einzelnen Schaltern mechanische Verriegelungen vorgesehen werden können. Aus Sicherheitsgründen und zur Vereinfachung der Bedienung ist jedoch eine vorzugsweise elektrische Steuerschalteinheit 20 vorgesehen, in der in üblicher Schaltungstechnik die einzelnen Funktionen miteinander verknüpft sind. Hierbei ist vorgesehen, daß der Niveaufühler 18 so geschaltet ist, daß sich das Gebläse 10 von Hand oder auch selbsttätig erst dann einschaltet, wenn das hierdurch vorgegebene Betriebsniveau erreicht ist. Ein vorzeitiges Einschalten des Gebläses ist somit ausgeschlossen.

Der zweite Niveaufühler 19, der in nur geringem Abstand über dem Wannenboden angeordnet ist, ist über die Steuerschaltung mit dem Stellantrieb 7ʹ des Absperrventils 7 verschaltet und zwar in der Weise, daß das Absperrventil 7 "stromlos offen" geschaltet ist und erst dann schließt, wenn beim Befüllen der Wanne der Wasserstand die Höhe des Niveaufühlers 19 erreicht hat. Hierdurch wird bewirkt, daß das in die Wanne laufende Frischwasser zunächst einmal über die Luftdüsen 3 und die Rohrleitung 4 in das Wannenabflußrohr 9 ein Teil des Frischwassers abläuft und so eine "Systemvorspülung" erzielt wird. Da die in der Regel durch die üblichen Badewannenarmaturen vorgegebenen Zulaufmengen erheblich größer sind als die durch den Gesamtquerschnitt der Düsen mögliche Ablaufmenge, ergibt sich, daß das Mindestniveau schon nach kurzer Zeit erreicht und das Absperrventil 7 geschlossen wird, so daß für die Systemvorspülung eine Menge von höchstens etwa 5 l Frischwasser verbraucht wird.

Wird nun durch Öffnen des Badewannenablaufs 9ʹ die Badewanne entleert, so wird selbst bei eingeschaltetem Gebläse 10 nach dem Unterschreiten des durch den Niveaufühler 18 vorgegebenen Betriebsniveaus das Gebläse 10 abgeschaltet. In der Steuereinheit 20 sind nun das Absperrventil 7, die Spülpumpe 16 und auch das Gebläse 10 über ein Zeitschaltwerk miteinander verschaltet, das über einen Steuerimpuls beim Unterschreiten des durch den Niveaufühler 19 vorgegebenen Mindestniveaus in Gang gesetzt wird. Das Absperrventil bleibt hierbei geschlossen, so daß zunächst einmal der gesamte Wanneninhalt ablaufen kann. Nach einer Zeitdauer t1, die so bemessen ist, daß die vollständige Entleerung der Wanne gewährleistet ist, wird die Spülpumpe 16 eingeschaltet, die während einer Zeitdauer t2 Spülflüssigkeit über die Zuleitung 11 in die Rohrleitung 4 drückt, die dann über die Düsen 3 in die Wanne einläuft und von dort über den Wannenablauf 9 abgezogen wird. Damit wird das Luftsystem "rückgespült". Durch den Rückflußverhinderer 6 wird die Zuleitung zum Gebläse 10 abgesperrt. Dann schaltet die Spülpumpe 16 ab, so daß für eine Zeitdauer t3 bei immer noch geschlossenem Absperrventil 7 die Spülflüssigkeit in der Rohrleitung 4 verbleibt, so daß die zugegebenen Desinfektionsmittel einwirken können und etwa noch vorhandene, mechanisch nicht ausgespülte Keime abtöten können. Nach Ablauf der Zeitdauer t3 wird das Absperrventil 7 für eine Zeitdauer t4 geöffnet, die so bemessen ist, daß die Rohrleitung 4 vollständig über das Wannenabflußrohr 9 entleert wird. Anschließend wird das Absperrventil 7 wieder geschlossen und nunmehr das Gebläse 10 auf einer geringen Leistungsstufe, d.h. mit niedriger Drehzahl eingeschaltet, so daß die Rohrleitung 4 nunmehr trocken geblasen werden kann. Zweckmäßigerweise ist hierzu dem Gebläse 10 druckseitig eine Zusatzheizung 21 vorgeschaltet, so daß das Trockenblasen mit aufgewärmter Luft erfolgen kann. Nach Ablauf der für das Trockenblasen vorgegebenen Zeitdauer t5 wird das Gebläse abgeschaltet und das Absperrventil geöffnet, so daß für die Zeit der Nichtbenutzung die Rohrleitung 4 über die Düsen und den Wannenablauf 9 belüftet bleibt.

Die "Technik" ist wie in der Zeichnung angedeutet, zweckmäßigerweise in einem Technikschrank zusammengeschlossen, der als fertige Baueinheit geliefert werden kann, so daß lediglich die erforderlichen Installationsanschlüsse vorzunehmen sind. Die Rohrverbindungen zwischen dem Technikschrank und der Wanne können auch über flexible Leitungen mit entsprechendem Querschnitt erfolgen, so daß auch eine Anordnung über Eck möglich ist. Es muß lediglich dafür Sorge getragen werden, daß zumindest alle flüssigkeitsführenden Leitungen durchgehend ein Gefälle in Richtung auf das Wannenablaufrohr 9 aufweisen und keine Durchbiegungen vorhanden sind, in denen sich Flüssigkeit ansammeln kann.

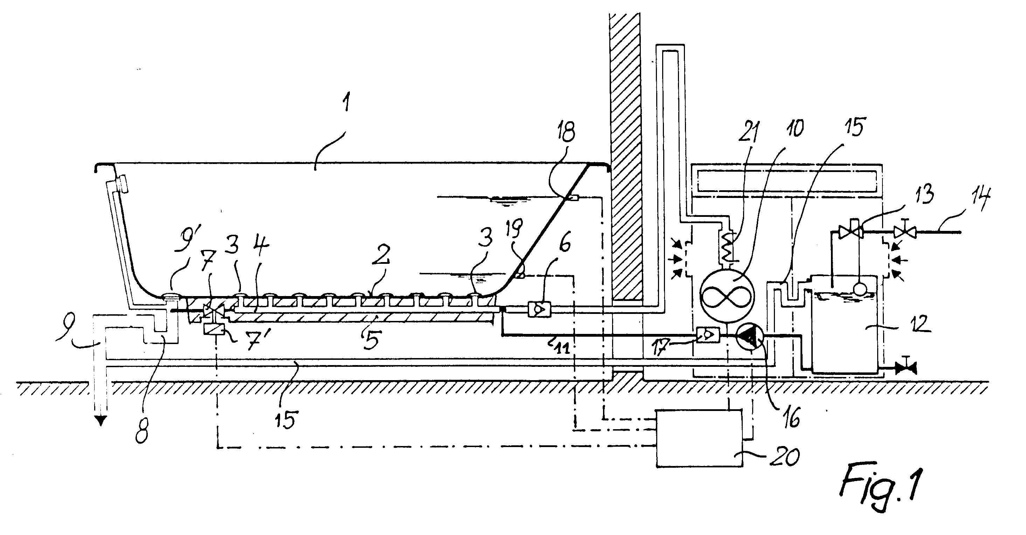

Da bei Wannen aus Kunststoff der Wannenkörper in üblicher Weise aus einer Acrylplatte tiefgezogen und anschliessend auf der Außenseite mit einer glasfaserverstärkten ausgehärteten Harzschicht überzogen ist, läßt sich die in Fig. 1 schematisch angedeutete Wannenbodenverstärkung 5 in besonders einfacher Weise auch dadurch herstellen, daß nach dem Bohren der Durchgangslöcher die Luftdüsen und die sie verbindenden Rohrleitungen montiert werden. Die Rohrleitungen verlaufen auf der Außenseite des Wannenbodens in Wannenlängsrichtung, so daß sich je nach Zahl der Düsenreihen im Wannenboden, in der Regel wenigstens zwei Düsenreihen, entsprechend zwei längslaufende Rohrleitungen auf der Wannenaußenseite ergeben. Zur Bildung der Wannenbodenverstärkung wird nun eine Verstärkungsplatte, beispielsweise eine Holzspanplatte, in den Zwischenraum zwischen den Rohrleitungen eingelegt, wobei der Rand der Verstärkungsplatte bis nahe an die Rohrleitungen heranreicht. Die von der Verstärkungsplatte überdeckte Fläche wird vorher noch mit einer als Kleber wirkenden Harzschicht versehen, so daß nach dem Aushärten dieses Harzes eine feste Verbindung zwischen der Verstärkungsplatte und dem Wannenkörper besteht. Nach dem Einlegen der Verstärkungsplatte werden nunmehr die Verstärkungsplatte und die Rohrleitungen mit einem gießbaren, aushärtenden Harz, das auch mit Füllstoffen versehen sein kann, übergossen, so daß die Verstärkungsplatte und die Rohrleitungen in die Harzschicht eingebettet werden. Im Bereich des Übergangs der Bodenfläche in die Seitenwandfläche wird vorher noch ein entsprechender Formrand aufgesetzt, so daß die aufgegossene Harzmenge die Rohrleitungen auch entlang ihrer Außenseiten vollständig umschließt. Gegenüber dem bisher üblichen Einlaminieren von Verstärkungsplatten durch Umspritzen mit glasfaserverstärktem Polyesterharz, besteht der Vorteil der vorstehend beschriebenen Anordnung darin, daß das Aufbringen der Verstärkung erheblich vereinfacht wird und auch die zwischen den Kanten der Verstärkungsplatte und den längslaufenden Rohrleitungen sowie zwischen den Rohrleitungen und dem Wannenboden zwischen den einzelnen Zuleitungsfittings bestehenden Hinterschneidungen zuverlässig ausgefüllt werden. Hierdurch ergibt sich nicht nur eine optisch einwandfreie Außenansicht der Wanne im Bodenbereich, sondern zugleich auch ein zusätzlicher Verstärkungseffekt durch die in die Harzmasse eingebetteten längslaufenden Rohrleitungen. Da die Rohrleitungen vollständig abgedeckt sind, kann eine derartige Wanne vollständig vorgefertigt dem Sanitärhandel bzw. dem Installateur zur Verfügung gestellt werden, wobei sichergestellt ist, daß während des Transportes oder bei der Lagerung die Rohrleitungen nicht beschädigt oder die Fixierung und Abdichtung zwischen den Düsen und den Rohrleitungen nicht gelockert werden kann.

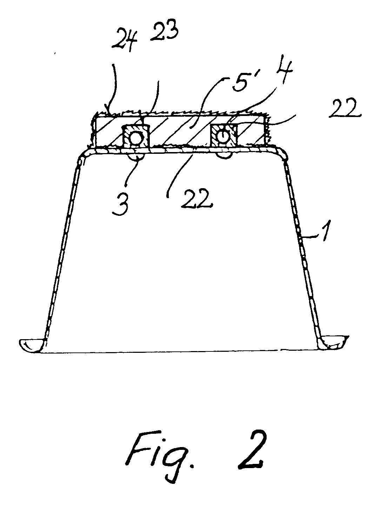

Eine besonders vorteilhafte Gestaltung ist in Fig. 2 dargestellt. Bei dieser Ausführungsform ist die aus einer Holzspanplatte hergestellte Verstärkungsplatte 5ʹ entsprechend der Zahl an Rohrleitungen 4 mit Längsnuten 23 versehen. Die Rohrleitungen 4 liegen bei entsprechend ausgebildeten Anschlußfittings für die Düsen 3 nahezu auf der Wannenaußenfläche auf, wobei für sogenannte Luftsprudelsysteme der Außendurchmesser beispielsweise nur 10 mm beträgt. Zur Montage werden die Längsnuten 23 mit Kleber 22 ausgefüllt und die der Wannenaußenfläche zugekehrte Fläche der Verstärkungsplatte ebenfalls mit Kleber 22 beschichtet. Dann wird die Verstärkungsplatte auf die Wannenaußenfläche aufgedrückt, wobei die Rohrleitungen 4 in die mit Kleber gefüllten Längsuten 23 eindringen. Beim Aufdrücken wird hierbei der Kleber auch in die Zwischenräume und Hinterschneidungen zwischen Rohrleitung und Wannenaußenfläche gepreßt, so daß die Rohrleitung in ihrer Längsnut vollständig in den Kleber eingebettet ist. Auf der Außenseite ist die Verstärkungsplatte 5ʹ mit einer Deckschicht 24, beispielsweise einer aufgespritzten Polyesterharzschicht, versehen, die auch deren Seitenkanten bis an die Wannenaußenfläche abdeckt und das Eindringen von Feuchtigkeit verhindert.

## Patentansprüche

1. Badewanne mit einem verschließbaren Wannenablauf (9'), der an ein Wannenablaufrohr (9) angeschlossen ist, und mit vorzugsweise im Bodenbereich eingebauten Düsen (3) für die Einleitung von Luft, mit denen die Druckseite eines Gebläses (10) über wenigstens eine Luftzuleitung verbunden ist, wobei in der Zuleitung (4) zwischen Gebläse (10) und der in Strömungsrichtung der Luft gesehen ersten Düse (3) ein Rückflußverhinderer (6) angeordnet ist, **dadurch gekennzeichnet,**
daß die Luftzuleitung als Rohrleitung ausgebildet und mit dem Wannenablaufrohr (9) verbunden ist und daß - bezogen auf die Luftbeaufschlagung - zwischen der letzten Düse (3) und der Einmündung in das Wannenablaufrohr in der Rohrleitung (4) ein mit einem unabhängig vom Wannenablauf steuerbaren Stellantrieb (7') versehenes Absperrventil (7) angeordnet ist, daß in die Rohrleitung (4) eine Zuleitung (11) für eine Spülflüssigkeit einmündet, die an eine Spülflüssigkeitsversorgung angeschlossen ist und daß in der Zuleitung (11) - in Strömungsrichtung gesehen - hinter der Einmündung der Spülflüssigkeitsversorgung ein Rückflußverhinderer (17) angeordnet ist.

2. Badewanne nach Anspruch 1, dadurch gekennzeichnet, daß als Versorgung ein Speicherbehälter (12) vorgesehen ist.

3. Badewanne nach Anspruch 1 oder 2, dadurch gekennzeichnet, daß in der vom Speicherbehälter ausgehenden Zuleitung (11) eine Spülpumpe (16) angeordnet ist.

4. Badewanne nach einem der Ansprüche 1 bis 3, dadurch gekennzeichnet, daß in Strömungsrichtung gesehen hinter der Spülpumpe (16) der Rückflußverhinderer (17) angeordnet ist.

5. Badewanne nach einem der Ansprüche 1 bis 4, dadurch gekennzeichnet, daß der Speicherbehälter (12) an eine Wasserzuleitung (14) angeschlossen ist, die über ein Schwimmerventil (13) absperrbar ist.

6. Badewanne nach Anspruch 5, dadurch gekennzeichnet, daß das Schwimmerventil (13) ein Spülkastenventil ist.

7. Badewanne nach einem der Ansprüche 1 bis 6, dadurch gekennzeichnet, daß der Speicherbehälter (12) mit einem Überlaufrohr (15) versehen ist, das an das Wannenablaufrohr (9) angeschlossen ist.

8. Badewanne nach einem der Ansprüche 1 bis 7, dadurch gekennzeichnet, daß an der Wand der Wanne (1) mit Abstand über dem Boden (2) ein Niveaufühler (18) angeordnet ist, der über eine Steuerschaltung mit dem Gebläse (10) verbunden ist, die ein Einschalten erst zuläßt, wenn das durch den Niveaufühler (18) vorgegebene Betriebsniveau erreicht ist.

9. Badewanne nach einem der Ansprüche 1 bis 8, dadurch gekennzeichnet, daß an der Wand der Wanne (1) unterhalb des ersten Niveaufühlers (18) ein zweiter Niveaufühler (19) mit geringem Abstand über dem Wannenboden (2) angeordnet ist, der über eine Steuerschaltung mit dem Stellantrieb (7ʹ) des Absperrventils (7) verbunden ist, die das Absperrventil (7) beim Füllen erst dann schließt, wenn das durch den zweiten Niveaufühler (19) vorgegebene Mindestniveau erreicht ist.

10. Badewanne nach einem der Ansprüche 1 bis 9, dadurch gekennzeichnet, daß das Absperrventil (7), die Spülpumpe (16) und das Gebläse (10) über ein Zeitschaltwerk in der Steuerschaltung (20) so miteinander verbunden sind, daß beim Entleeren der Wanne (1) bei Unterschreitung des Mindestniveaus bei geschlossenem Absperrventil (7) nach Ablauf einer Zeitdauer t1 die Spülpumpe (16) für eine Zeitdauer t2 eingeschaltet und daß nach Ablauf der Zeitdauer t2 während einer Zeitdauer t3 bei geschlossenem Absperrventil (7) die Spülpumpe (16) abgeschaltet bleibt, daß nach Ablauf der Zeitdauer t3 das Absperrventil (7) öffnet und nach Ablauf einer Zeitdauer t4 wieder schließt und das Gebläse (10) mit geringer Leistung einschaltet und daß nach Ablauf einer Zeitdauer t5 das Gebläse (10) abschaltet und das Absperrventil (7) öffnet.

11. Badewanne aus Kunststoff, insbesondere aus Acryl mit einer Wannenbodenverstärkung nach einem der Ansprüche 1 bis 10, dadurch gekennzeichnet, daß die mit den Luftdüsen versehene Rohrleitung (4) im Bereich des Wannenbodens wenigstens teilweise in das Material der Wannenbodenverstärkung (5) eingebettet ist.

12. Badewanne nach Anspruch 11, dadurch gekennzeichnet, daß die Wannenbodenverstärkung (5) durch wenigstens eine Verstärkungsplatte, vorzugsweise aus einem Holzwerkstoff, gebildet wird, die mit der Wannenaußenfläche verklebt ist, daß die Rohrleitung (4ʹ) an deren Rand entlang verläuft und daß die Verstärkungsplatte mit der Rohrleitung in einer geschlossenen Schicht aus einem gießbaren aushärtenden Harz eingebettet ist, die im Randbereich umlaufend mit der Wannenaußenfläche verbunden ist.

13. Badewanne nach Anspruch 11, dadurch gekennzeichnet, daß die Verstärkungsplatte (5ʹ) auf ihrer der Wannenaußenfläche zugekehrten Seite mit jeweils einer Nut (23) zur Aufnahme der Rohrleitung (4) versehen ist, die mit Kleber (22) ausgefüllt ist, der nach dem Andrücken der Verstärkungsplatte (5ʹ) an die Wannenaußenfläche die Rohrleitung (4) umschließt.

14. Badewanne nach Anspruch 11 oder 12, dadurch gekennzeichnet, daß die Wannenbodenverstärkung (5) durch ein vorzugsweise mit Füllstoffen vermischtes gießbares aushärtendes Harz, vorzugsweise ungesättigtes Polyesterharz, gebildet wird.

15. Badewanne nach Anspruch 11, 13 oder 14, dadurch gekennzeichnet, daß die Wannenbodenverstärkung durch ein vorgefertigtes Formteil aus mit Füllstoffen versetztem Harz gebildet wird, in das die Zuleitungsfittings (10) und die zugehörige Rohrleitung (4) eingegossen sind und das vorzugsweise durch Klebung mit der Außenfläche des Wannenkörpers fest verbunden ist.

## Claims

1. A bath with a closable bath outlet (9') which is connected to a bath waste pipe (9) and with nozzles (3), preferably installed in the base region, for introducing air, to which the pressure side of a fan (10) is connected by means of at least one air feed line, with a backflow prevention means (6) being located in the feed line (4) between the fan (10) and the first nozzle (3) viewed in the direction of flow of the air,
characterised in that the air feed line is designed as a pipe and is connected to the bath waste pipe (9) and that - relative to the action of the air - a stop valve (7) provided with an actuating drive (7') which can be controlled independently of the bath outlet is located in the pipe (4) between the final nozzle (3) and the opening into the bath waste pipe, that a feed pipe (11) for a rinsing liquid opens into the pipe (4), which pipe is connected to a rinsing liquid supply means and that a backflow prevention means (17) is located behind the opening of the rinsing liquid supply means in the feed pipe (11), viewed in the direction of flow.

2. A bath according to Claim 1, characterised in that a storage tank (12) is provided as the supply means.

3. A bath according to Claim 1 or 2, characterised in that a flushing pump (16) is provided in the feed line (11) which leaves the supply means.

4. A bath according to one of Claims 1 to 3, characterised in that, viewed in the direction of flow, the backflow prevention means (17) is located after the flushing pump (16).

5. A bath according to one of Claims 1 to 4, characterised in that the storage tank (12) is connected to a water feed pipe (14) which can be closed off by means of a float valve (13).

6. A bath according to Claim 5, characterised in that the float valve (13) is a flushing tank valve.

7. A bath according to one of Claims 1 to 6, characterised in that the storage tank (12) is provided with an overflow pipe (15) which is connected to the bath waste pipe (9).

8. A bath according to one of Claims 1 to 7, characterised in that a level sensor (18) is provided on the wall of the bath (1) at a distance from the base (2), which sensor is connected to the fan (10) by means of a control circuit which does not permit switching-on until the operating level set by the level sensor (18) is reached.

9. A bath according to one of Claims 1 to 8, characterised in that a second level sensor (19) is located on the wall of the bath (1) beneath the first level sensor (18) at a short distance above the base (2) of the bath, which second sensor is connected by means of a control circuit to the actuating drive (7') of the stop valve (7), which circuit does not close the stop valve (7) during filling until the minimum level set by the second level sensor (19) is reached.

10. A bath according to one of Claims 1 to 9, characterised in that the stop valve (7), the flushing pump (16) and the fan (10) are interconnected by means of a time-switch in the control circuit (20) such that, when the bath (1) is emptied, when the level drops below the minimum level when the stop valve (7) is closed after a period t1 has elapsed the flushing pump (16) is switched on for a period t2, and that once the period t2 has elapsed the flushing pump (16) remains shut off for a period t3 with the stop valve (7) closed, that once the period t3 has elapsed the stop valve (7) opens and closes again once a period t4 has elapsed, and switches on the fan (10) with low power and that once a period t5 has elapsed switches off the fan (10) and opens the stop valve (7).

11. A bath made of plastic, in particular of acrylic, with a bath base reinforcement according to one of Claims 1 to 10, characterised in that the pipe (4) provided with the air nozzles is at least partially embedded in the material of the bath base reinforcement (5) in the region of the bath base.

12. A bath according to Claim 11, characterised in that the bath base reinforcement (5) is formed by at least one reinforcing plate, preferably of a timber material, which is glued to the outer surface of the bath, that the pipe (4') runs along the edge thereof and that the reinforcing plate with the pipe is embedded in a closed layer of a pourable hardening resin, which is joined on the periphery to the outer bath surface in the edge region.

13. A bath according to Claim 11, characterised in that the reinforcing plate (5') is provided on its side facing the outer bath surface with one groove (23) for receiving the pipe (4) in each case, which groove is filled with adhesive (22) which after the reinforcing plate (5') has been pressed on to the outer bath surface surrounds the pipe (4).

14. A bath according to Claim 11 or 12, characterised in that the bath base reinforcement (5) is formed by a pourable hardening resin preferably mixed with fillers, preferably unsaturated polyester resin.

15. A bath according to Claim 11, 13 or 14, characterised in that the bath base reinforcement is formed by a prefabricated shaped part made of resin with fillers added, into which the feed pipe fittings (10) and the associated pipe (4) are cast and which is securely connected to the outer surface of the bath body, preferably by gluing.

## Revendications

1. Baignoire à orifice d'écoulement de baignoire (9') obturable raccordé à un tuyau d'écoulement de baignoire (9) et à buses (3), de préférence incorporées dans la zone du fond, pour l'introduction d'air, auxquelles est raccordé, par au moins une conduite d'amenée d'air, le côté de refoulement d'une soufflante (10), un dispositif anti-reflux (6) étant monté dans la conduite d'amenée (4) entre la soufflante (10) et la première buse (3), vu dans le sens de circulation de l'air, caractérisée en ce que la conduite d'amenée d'air se présente sous forme d'une tuyauterie et est connectée au tuyau d'écoulement de baignoire (9) et en ce que - par rapport à l'admission d'air - entre la dernière buse (3) et l'embouchure dans le tuyau d'évacuation de baignoire est disposée, dans la tuyauterie (4), une soupape d'arrêt (7) pourvue d'un entraînement de réglage (7') pouvant être commandé indépendamment de l'orifice d'évacuation de la baignoire, que dans la tuyauterie (4) aboutit une conduite d'amenée (11) d'un liquide de rinçage qui est raccordée à une alimentation en liquide de rinçage et que dans la conduite d'amenée (11) - vu dans le sens de circulation - est disposé, derrière l'embouchure de l'alimentation en liquide de rinçage, un dispositif anti-reflux (17).

2. Baignoire suivant la revendication 1, caractérisée en ce qu'un réservoir (12) est prévu comme alimentation.

3. Baignoire suivant la revendication 1 ou 2, caractérisée en ce que dans la conduite d'amenée (11) partant du réservoir est disposée une pompe de rinçage (16).

4. Baignoire suivant l'une des revendications 1 à 3, caractérisée en ce que, vu dans le sens de circulation, le dispositif anti-reflux (17) est placé derrière la pompe de rinçage (16).

5. Baignoire suivant l'une des revendications 1 à 4, caractérisée en ce que le réservoir (12) est raccordé à une conduite d'amenée d'eau (14) qui peut être fermée par une soupape à flotteur (13).

6. Baignoire suivant la revendication 5, caractérisée en ce que la soupape à flotteur (13) est une soupape de bac de rinçage.

7. Baignoire suivant l'une de revendications 1 à 6, caractérisée en ce que le réservoir (12) est pourvu d'un tuyau de trop-plein (15) qui est raccordé au tuyau d'écoulement de baignoire (9).

8. Baignoire suivant l'une de revendications 1 à 7, caractérisée en ce que sur la paroi de la baignoire (1) est disposé, à distance au-dessus du fond (2), un détecteur de niveau (18) qui est relié à la soufflante (10) par un circuit de commande qui ne permet un enclenchement que lorsque le niveau de service indiqué par le détecteur de niveau (18) est atteint.

9. Baignoire selon l'une des revendications 1 à 8, caractérisée en ce qu'un deuxième détecteur de niveau (19) est disposé sur la paroi de la baignoire (1) au-dessous du premier détecteur de niveau (18), à une distance réduite au-dessus du fond (2) de la baignoire, lequel deuxième détecteur de niveau est relié par un circuit de commande à l'entraînement de réglage (7') de la soupape d'arrêt (7), lequel circuit ferme la soupape d'arrêt (7) lors du remplissage, seulement lorsque le niveau minimum prédéterminé par le deuxième détecteur de niveau (19) est atteint.

10. Baignoire suivant l'une des revendications 1 à 9, caractérisée en ce que la soupape d'arrêt (7), la pompe de rinçage (16) et la soufflante (10) sont reliées l'une à l'autre, par une minuterie dans le circuit de commande, de telle manière que, lors du vidage de la baignoire (1), lorsque, à soupape d'arrêt (7) fermée, le niveau passe au-dessous du niveau minimum, après écoulement d'un laps de temps t1, la pompe de rinçage (16) est enclenchée pendant un laps de temps t2 et que, après écoulement du laps de temps t2, la pompe de rinçage (16) est arrêtée pendant un laps de temps t3, à soupape d'arrêt (7) fermée, qu'après écoulement du laps de temps t3, la soupape d'arrêt (7) s'ouvre et après écoulement d'un laps de temps t4 se referme et la soufflante (10) s'enclenche avec une faible puissance et qu'après écoulement d'un laps de temps t5, la soufflante (10) est arrêtée et la soupape d'arrêt (7) s'ouvre.

11. Baignoire en matière plastique, en particulier en acrylique, à renforcement du fond de baignoire, suivant l'une des revendications 1 à 10, caractérisée en ce que la tuyauterie (4) pourvue des buses est, dans la zone du fond de baignoire, au moins partiellement encastrée dans le matériau de renforcement du fond de baignoire (5).

12. Baignoire suivant la revendication 11, caractérisée en ce que le renforcement du fond de baignoire (5) est formé par au moins une plaque de renforcement, de préférence en un matériau à base de bois, qui est assemblée par collage à la face extérieure de la baignoire, que la tuyauterie (4') s'étend le long de son bord et que la plaque de renforcement est, avec la tuyauterie, encastrée dans une couche fermée réalisée en une résine durcissable moulable qui est assemblée en périphérie, dans la zone de bordure, avec la face extérieure de la baignoire.

13. Baignoire suivant la revendication 11, caractérisée en ce que la plaque de renforcement (5') est munie, sur sa face tournée vers la face extérieure de la baignoire, respectivement, d'une rainure (23), destinée à recevoir la tuyauterie (4), qui est remplie d'un adhésif (22) qui, après pression de la plaque de renforcement (5') contre la face extérieure de la baignoire, entoure la tuyauterie (4).

14. Baignoire suivant la revendication 11 ou 12, caractérisée en ce que le renforcement du fond de baignoire (5) est formé par une résine durcissable moulable, de préférence mélangée avec des charges, de préférence une résine polyester non-saturée.

15. Baignoire suivant la revendication 11, 13 ou 14, caractérisée en ce que le renforcement du fond de baignoire (5) est formé par une pièce moulée préfabriquée, réalisée en une résine mélangée à des charges, dans laquelle sont coulés les raccords de la conduite d'amenée (10) et la tuyauterie (4) y afférente, et qui est rendue solidaire de la face extérieure du corps de baignoire, de préférence par collage.
